# EUROPEAN PATENT SPECIFICATION

(11) **EP 2 626 112 B1**
(45) Date of publication and mention of the grant of the patent: **24.09.2014**
(21) Application number: 13154028.8
(22) Date of filing: 05.02.2013
(51) Int. Cl.: A61P 27/06, A61K 36/906

(54) **A plant extract from the genus lindera for lowering intraocular pressure**
Pflanzenextrakt der Gattung Lindera zur Senkung des Augeninnendrucks
Extrait de plante du genre lindera pour abaisser la pression intraoculaire

(30) Priority: 10.02.2012 KR 20120013703
(43) Date of publication of application: 14.08.2013
(73) Proprietor: Korea Institute of Science and Technology, Seoul 136-791 (KR)
(72) Inventor: Jung, Sang Hoon, Seoul 138-220 (KR); Jo, Hyoung, Gangwon-do 210-791 (KR); Kim, Chul Young, Seoul 151-050 (KR); Choi, Soon Jung, 210-952 Gangwon-do (KR); Lee, Eun Ha, Gyeonggi-do 487-829 (KR); Kim, Kyung A, Gangwon-do 210-778 (KR)
(74) Representative: advotec.

(56) References cited:
- EP-A2- 2 510 933
- US-A1- 2011 045 111
- WANG HAOGUI: "A topical medicament of Herba Vaccinii Urophylli / A topical pharmaceutical composition of treatment of Herba Vaccinii Urophylli for treating rheumatic arthralgia, traumatic blood stasis and pain, and soft tissue contusion, ect", TCM,, 1 March 2006 (2006-03-01), XP002628397,

## Description

### [Technical Field]

The present disclosure relates to a composition containing an extract of a plant in the genus *Lindera* for use in lowering increased intraocular pressure and/or treating glaucoma.

### [Background Art]

Since the cornea, lens, vitreous body, etc. of the eye lacks blood vessels, aqueous humor provides nutrients and carries waste materials. If the aqueous humor is produced normally but is not drained normally, the fluid pressure inside the eye is increased. The increased intraocular pressure restricts blood flow to the eye and exerts pressure continuously to the optic nerve, leading to fatal damage to the optic nerve and loss of vision and visual field. Glaucoma is a disease in which the optic nerve is compressed or damaged owing to increased intraocular pressure or restricted blood flow, ultimately leading to loss of vision. Glaucoma occurs with a frequency of 0.5-2% in adults who are 40 years or older. It is characterized by raised intraocular pressure above the normal value of 15-20 mmHg. As glaucoma develops, the inside of the pupil looks green as the intraocular pressure increases.

At present, medication or surgery for preventing the increase of intraocular pressure is the mainstay of glaucoma treatment. But, since perfect cure is impossible, control of intraocular pressure via pharmaceuticals, laser, surgery, etc. is necessary to minimize optic nerve damage.

The inventors of the present disclosure have made efforts to solve this problem. As a result, they have found out that administration of an extract of a plant in the genus *Lindera* to the eye is effective in lowering or controlling the increase of intraocular pressure.

### [Disclosure]

The present invention is defined by the claims.

### [Technical Problem]

The present disclosure is directed to providing a composition containing a substance that controls intraocular pressure.

### [Technical Solution]

In a general aspect, there is provided a composition for lowering intraocular pressure containing an extract of a plant in the genus *Lindera* as an active ingredient.

### [Advantageous Effects]

The composition of the present disclosure is useful since it allows control of intraocular pressure. Further, the composition of the present disclosure is advantageous in that it has fewer side effects than chemical substances and is environmentally friendly since the extract derived from the natural product is used to control the intraocular pressure. In addition, since the extract contained in the composition of the present disclosure can be obtained from the plants in the genus *Lindera* which are easily available, it can be usefully used economically and commercially.

### [Description of Drawings]

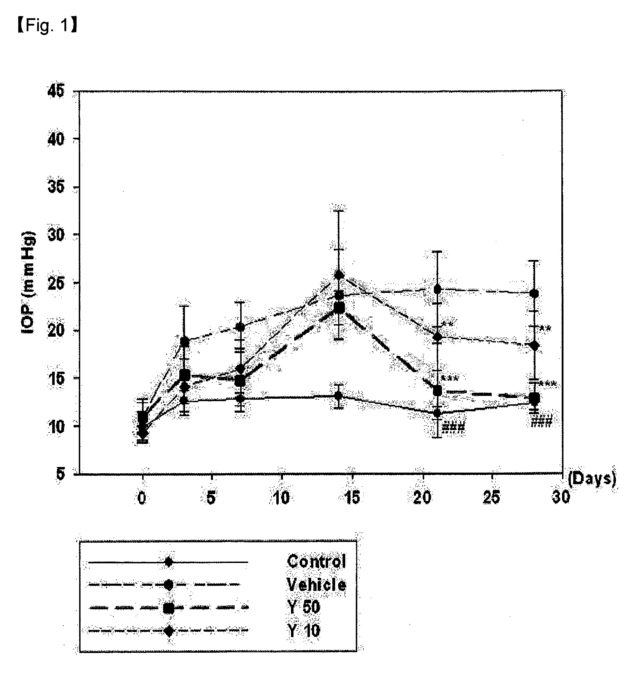

Fig. 1 shows a result of measuring change in intraocular pressure after inducing increase of the intraocular pressure using silicone oil and then intraabdominally injecting a *Lindera obtusiloba* extract.

### [Best Mode]

The present disclosure relates to a composition for lowering intraocular pressure containing an extract of a plant in the genus *Lindera* as an active ingredient.

In the composition for lowering intraocular pressure according to the present disclosure, the plant in the genus *Lindera* may include *Lindera erythrocarpa, Lindera obtusiloba, Lindera sericea* and *Lindera glauca.*

In the composition for lowering intraocular pressure according to the present disclosure, the plant in the genus *Lindera* may be *Lindera obtusiloba.* The *Lindera obtusiloba* may be one cultivated, picked or commercially available, without any limitation. The extract may be obtained from the whole plant of the *Lindera obtusiloba,* specifically from leaves, stem or root, without being limited thereto.

The composition for lowering intraocular pressure according to the present disclosure may be used to prevent or treat glaucoma.

The composition for lowering intraocular pressure according to the present disclosure may contain 0.1-50 wt% of the extract based on the total weight of the composition. If the extract is contained in an amount less than 0.1% of the total weight of the composition, the effect of lowering intraocular pressure is slight. And, if it is contained in an amount exceeding 50%, the intraocular pressure becomes lower than the normal level. Accordingly, the extract may be contained in an amount of 0.5-45 wt%, 1-40 wt%, 1.5-35 wt%, 2-30 wt%, 2.5-25 wt%, 3-20 wt%, 3.5-15 wt%, 4-10 wt% or 4.5-10 wt% based on the total weight of the composition.

Specifically, the extract of the plant in the genus *Lindera* may be prepared as follows, but the present disclosure is not limited thereto.
1) Extraction of a dried plant in the genus *Lindera* using an extraction solvent.
2) Filtration of the extract obtained in 1).
3) Concentration of the extract filtered in 2) under reduced pressure to prepare the desired extract.

The extract may be obtained using a method commonly employed in the art, using apparatuses such as supercritical fluid extraction, subcritical fluid extraction, high-temperature extraction, high-pressure extraction or ultrasonic extraction or using an absorbent resin such as XAD or HP-20. Specifically, the extraction may be carried out by refluxing at elevated temperatures or room temperature, but the present disclosure is not limited thereto. The extraction may be carried out 1-5 times, specifically 3 times, but the present disclosure is not limited thereto.

Specifically, the concentration under reduced pressure in 3) may be performed using a rotary vacuum evaporator, but the present disclosure is not limited thereto. And, the drying may be performed by drying under reduced pressure, vacuum drying, superheated steam drying, spray drying, natural air drying or freeze drying, but the present disclosure is not limited thereto.

In the composition for lowering intraocular pressure according to the present disclosure, the extract may be obtained using an extraction solvent including water, alcohol or a mixture thereof. In the composition for lowering intraocular pressure according to the present disclosure, the alcohol may include a C₁-C₄ alcohol. And, in the composition for lowering intraocular pressure according to the present disclosure, the alcohol may be methanol or ethanol. In the present disclosure, the extraction solvent may be used in an amount corresponding to 1-20 times, specifically 10 times, the dry weight of the plant in the genus *Lindera,* but the present disclosure is not limited thereto. The extraction may be performed by hot water extraction, immersion extraction, reflux condensation extraction, ultrasonic extraction, or the like. Specifically, the extraction may be performed 1-5 times by ultrasonic extraction. Extraction temperature may be specifically 10-100 °C, more specifically room temperature, but the present disclosure is not limited thereto. Extraction time may be specifically 1-7 days, more specifically 3-7 days, but the present disclosure is not limited thereto.

The composition for lowering intraocular pressure according to the present disclosure may be a pharmaceutical composition. The pharmaceutical composition may contain 30-80 mg/kg of the extract of the plant in the genus *Lindera* based on the total weight of the pharmaceutical composition. If the extract of the plant in the genus *Lindera* is contained in an amount less than 30 mg/kg, the intraocular pressure is not lowered to the normal level. And, an amount exceeding 80 mg/kg is not appropriate when considering the contents of other ingredients contained in the pharmaceutical composition. In this aspect, the pharmaceutical composition may contain 33-78 mg/kg, 35-76 mg/kg, 37-74 mg/kg, 40-72 mg/kg, 43-70 mg/kg, 45-68 mg/kg, 47-66 mg/kg, 50-64 mg/kg or 52-62 mg/kg of the extract of the plant in the genus *Lindera.* And, the pharmaceutical composition may be administered to a subject 2-3 times a week.

In an exemplary embodiment, the composition may be for preventing or treating glaucoma. It may be a pharmaceutical composition effective for preventing, alleviating or treating diseases caused by increased intraocular pressure. The pharmaceutical composition according to the present disclosure has an effect of preventing increase of intraocular pressure or lowering elevated intraocular pressure down to a normal level (15-20 mmHg).

When the composition according to the present disclosure is used for medical purposes, the composition may be formulated for oral or parenteral administration into solid, semisolid or liquid form by adding a commonly used organic or inorganic carrier to the active ingredient.

Formulations for oral administration may include tablet, pill, granule, hard/soft capsule, powder, fine granule, dust, emulsion, syrup, pellet, etc. Formulations for parenteral administration may include injection, medicinal drop, liquid, ointment, lotion, spray, suspension, emulsion, suppository, etc. The formulations may be prepared easily according to a known method using a surfactant, an excipient, a colorant, a fragrance, a preservative, a stabilizer, a buffer, a suspending agent or other commonly employed adjuvants.

The administration dosage of the active ingredient will vary depending on the age, sex and body weight of a subject, the particular disease or pathological condition to be treated, the severity of the disease or pathological condition, the route of administration and the discretion of a diagnoser. The determination of the administration dosage based on these factors is within the level of those skilled in the art. A general administration dosage is 0.001-2000 mg/kg/day, more specifically 0.5-1500 mg/kg/day.

The composition of the present disclosure may further contain a carrier, an excipient and a diluent appropriate to be used in the preparation of pharmaceuticals.

The composition according to the present disclosure may be formulated into powder, granule, tablet, capsule, suspension, emulsion, syrup, aerosol, etc. for oral administration or into formulation for external application, suppository or sterile solution for injection according to a known method. The carrier, excipient and diluent that can be contained in the composition of the present disclosure may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. For the formulation, a commonly used diluent or excipient such as a filler, an extender, a filler, a wetting agent, a disintegrator, a surfactant, etc. is used. Solid formulations for oral administration include tablet, pill, powder, granule, capsule, etc. Those solid formulations are prepared by mixing at least one excipient, e.g. starch, calcium carbonate, sucrose, lactose, gelatin, etc. with the composition of the present disclosure. Also, a lubricant such as magnesium stearate or talc may be included in addition to the excipient. Liquid formulations for oral administration include suspension, solution, emulsion, syrup, etc. These formulations may contain various excipients such as a wetting agent, a sweetener, an aromatic, a preservative, etc. in addition to general simple diluents such as water and liquid paraffin. Formulations for parenteral administration include sterilized aqueous solution, non-aqueous formulation, suspension, emulsion, freeze-dried formulation and suppository. The non-aqueous formulation or suspension may contain propylene glycol, polyethylene glycol, vegetable oil such as olive oil, injectable ester such as ethyl oleate, or the like. Witepsol, Macrogol, Tween 61, cocoa butter, laurin butter, glycerogelatin, etc. may be used as a base of the suppository.

The composition for lowering intraocular pressure according to the present disclosure may be a health food composition.

In an exemplary embodiment, the present disclosure provides a food additive containing the composition according to the present invention. In another instance of the present disclosure, a functional food or a health food containing the composition according to the present disclosure is disclosed. The composition may be for preventing or treating glaucoma. It may also be a composition effective for preventing, alleviating or treating diseases caused by increased intraocular pressure. The composition according to the present disclosure has an effect of preventing increase of intraocular pressure or lowering elevated intraocular pressure down to a normal level (15-20 mmHg).

The present disclosure provides various forms of food additive or functional food containing the composition according to the present disclosure. The composition may be processed into fermented milk, cheese, yogurt, juice, probiotic, dietary supplement, etc. and various other food additives. The food type is not particularly limited in the present disclosure. Examples of food that may contain the composition include drink, meat, sausage, bread, biscuit, rice cake, chocolate, candy, snack, pizza, instant and other noodles, gum, dairy products including ice cream, soup, beverage, alcoholic drink, vitamin complex, etc. and include all types of health food.

In one instance, the composition may contain other ingredients that can provide a synergic effect within a range not sacrificing the main effect intended by the present disclosure. For example, additives such as fragrance, pigment, sterilizer, antioxidant, antiseptic, moisturizer, thickener, mineral, emulsifier, synthetic polymer material, etc. may be further included for improvement of physical properties. Besides, auxiliary ingredients such as water-soluble vitamin, oil-soluble vitamin, polypeptide, polysaccharide, seaweed extract, etc. may be further included. Those skilled in the art may select these ingredients without difficulty considering particular formulation or purpose of use and the addition amount may be selected within a range not negatively affecting the purpose and effect desired in the present disclosure. For example, these ingredients may be added in an amount of 0.01-5 wt%, more specifically 0.01-3 wt%, based on the total weight of the composition.

The composition according to the present disclosure may be in various forms, including solution, emulsion, viscous mixture, tablet, powder, etc., and may be administered in various ways, including simple drinking, injection, spraying, squeezing, or the like.

A functional health drink composition according to the present disclosure may further include other ingredients in addition to the extract as an essential ingredient, without particular limitation. Like other general drinks, it may contain various flavors or natural carbohydrates. Examples of the natural carbohydrate include monosaccharides such as glucose, fructose, etc., disaccharides such as maltose, sucrose, etc., polysaccharides such as dextrin, cyclodextrin, etc. sugars and sugar alcohols such as xylitol, sorbitol, erythritol, etc. As the flavor, a natural flavor [thaumatin or stevia extract (e.g. rebaudioside A, glycyrrhizin, etc.)] or a synthetic flavor (saccharin, aspartame, etc.) may be used. The natural carbohydrate may be used in an amount of about 1-20 g, specifically about 5-12 g, per 100 mL of the composition of the present disclosure.

In addition, the composition according to the present disclosure may further contain various nutrients, vitamins, minerals (electrolytes), synthetic or natural flavors, colorants and extenders (cheese, chocolate, etc.), pectic acid and salts thereof, alginic acid and salts thereof, organic acids, protective colloidal viscofiers, pH regulators, stabilizers, antiseptics, glycerin, alcohols, carbonators used in carbonated drinks, or the like. In addition, the composition according to the present disclosure may further contain pulp for preparing fruit juice or vegetable juice. These ingredients may be used independently or in combination. The addition amount of these additives is of no great importance. It is usually selected within 0.1-20 parts by weight per 100 parts by weight of the extract of the plant in the genus *Lindera.*

The present disclosure also provides a method for lowering intraocular pressure by administering the composition for lowering intraocular pressure, the composition for lowering intraocular pressure or the health food additive composition lowering intraocular pressure to a subject.

### [Mode for Invention]

Hereinafter, the present disclosure will be described in detail through examples.

### [Example 1] Preparation of Lindera obtusiloba extract

The whole plant of dried *Lindera obtusiloba* was cut finely. After adding 2 L of ethanol to 13.48 kg of the cut *Lindera obtusiloba,* followed by reflux extraction for 3 hours while heating, the resulting extract was filtered. After the filtering, 2 L of ethanol was added again to the residue and reflux extraction was carried out for 3 hours while heating. This procedure was repeated twice and 6 L of extract was obtained. 6 L of the extract was concentrated at 35 °C under reduced pressure to obtain 1.1 kg of an ethanol extract. The extract and fractions thereof were dissolved in sterilized distilled water for further experiment.

### [Test Example 1] Lowering of intraocular pressure by Lindera obtusiloba extract

A laboratory rat was anesthetized by intramuscularly injecting 1 mL/kg of a mixture of 2% Rompun injection and Zoletil. Then, silicone oil (50 µL/eye) was injected into the vitreous body through the pars plana using a sterilized Hamilton equipped with a 33-G needle. After the injection, a drop of 0.5% Vigamox (Alcon Laboratories, Inc.) was dropped to prevent infection. For control, the same volume of 0.01 M PBS was injected. Silicone oil was injected into the left eye and the same volume of PBS was injected into the vitreous body of the right eye as control. Intraocular pressure was measured on days 0, 3, 7, 14, 21 and 28 using TonoLab. After the intraocular pressure was raised, the *Lindera obtusiloba* extract was intraabdominally injected once in 2 days.

It was confirmed that the intraocular pressure was raised by the injection of silicone oil and was lowered when the *Lindera obtusiloba* extract was intraabdominally injected (Fig. 1).

The composition containing the extract of a plant in the genus *Lindera* according to the present disclosure may be prepared into various formulations exemplified below, but the present disclosure is not limited thereto.

### [Formulation Example 1] Preparation of ophthalmic gel

| | |
|---|---|
| *Lindera obtusiloba* extract | 5 mg |
| Carbopol 934 | 20 mg |
| Triethylamine | adequate |
| Methyl parahydroxybenzoate | 2 mg |
| Sterilized purified water | ad. 1 g |

Methyl parahydroxybenzoate was dissolved in sterilized purified water by heating. After cooling, the *Lindera obtusiloba* extract was dissolved therein. After adding Carbopol 934 and dispersing by mixing with a high-speed mixer, the resultant was allowed to stand to remove air. Then, triethylamine was added dropwise while taking caution such that air was not included.

### [Formulation Example 2] Preparation of ophthalmic solution

| | |
|---|---|
| *Lindera obtusiloba* extract | 5 g |
| Benzalkonium chloride | 0.1 g |
| Sodium chloride | 5 g |
| Boric acid | 6.2 g |
| Tyloxapol | 1.0 g |
| Dilute hydrochloric acid | adequate |
| Sterilized purified water | ad. 1000 mL |

Sodium chloride and boric acid were sequentially added to and dissolved in the *Lindera obtusiloba* extract. The mixture was stirred after adding benzalkonium chloride and tyloxapol dissolved in a small volume of sterilized purified water thereto. After adjusting pH by adding dilute hydrochloric acid, the resultant was sterilized by filtering through 0.45-µm filter.

### [Formulation Example 3] Preparation of ophthalmic ointment

| | |
|---|---|
| *Lindera obtusiloba* extract | 5 mg |
| Sterilized purified water | 10 mg |
| Methyl parahydroxybenzoate | 2 mg |
| Anhydrous lanolin | 100 mg |
| White petrolatum | ad. 1 g |

The *Lindera obtusiloba* extract and methyl parahydroxybenzoate were added to a sterilized glass bowl. After dissolving the *Lindera obtusiloba* extract by adding sterilized purified water, the resultant was blended while adding anhydrous lanolin until a homogenous mixture was obtained.

### [Formulation Example 4] Preparation of soft capsule

330 mg of the *Lindera obtusiloba* extract, 50 mg of red ginseng extract, 2 mg of palm oil, 8 mg of hydrogenated palm oil, 4 mg of yellow beeswax and 6 mg of lecithin were mixed. 400 mg of the mixture was filled per capsule according to a commonly employed method.

### [Formulation Example 5] Preparation of tablet

150 mg of the *Lindera obtusiloba* extract, 100 mg of glucose, 50 mg of red ginseng extract, 96 mg of starch and 4 mg of magnesium stearate were mixed and 40 mg of 30% ethanol was added to form granules. After drying at 60 °C, a tablet was prepared using a tablet making machine.

### [Formulation Example 6] Preparation of granule

150 mg of the *Lindera obtusiloba* extract, 100 mg of glucose, 50 mg of red ginseng extract and 600 mg of starch were mixed and 100 mg of 30% ethanol was added to form granules. After drying at 60 °C, the granules were filled in a pouch. The final weight was 1 g per pouch.

### [Formulation Example 7] Preparation of drink

150 mg of the *Lindera obtusiloba* extract, 10 g of glucose, 50 mg of red ginseng extract, 2 g of citric acid and 187.8 g of purified water were mixed and filled in a bottle. The final volume was 200 mL per bottle.

### [Formulation Example 8] Preparation of health food

| | |
|---|---|
| *Lindera obtusiloba* extract | 1000 mg |

| Vitamin mixture | |
|---|---|
| Vitamin A acetate | 70 µg |
| Vitamin E | 1.0 mg |
| Vitamin B₁ | 0.13 mg |
| Vitamin B₂ | 0.15 mg |
| Vitamin B₆ | 0.5 mg |
| Vitamin B₁₂ | 0.2 µg |
| Vitamin C | 10 mg |
| Biotin | 10 µg |
| Nicotinamide | 1.7 mg |
| Folic acid | 50 µg |
| Calcium pantothenate | 0.5 mg |

| Mineral mixture | |
|---|---|
| Ferrous sulfate | 1.75 mg |
| Zinc oxide | 0.82 mg |
| Magnesium carbonate | 25.3 mg |
| Potassium phosphate monobasic | 15 mg |
| Calcium phosphate dibasic | 55 mg |
| Potassium citrate | 90 mg |
| Calcium carbonate | 100 mg |
| Magnesium chloride | 24.8 mg |

The above-described composition of vitamin and mineral mixtures is given as a specific example relatively adequate for health food. However, the composition may be changed variously. The above ingredients may be mixed and prepared into granule accordingly to a commonly employed health food preparation method.

### [Formulation Example 9] Preparation of health drink

| | |
|---|---|
| *Lindera obtusiloba* extract | 1000 mg |
| Citric acid | 1000 mg |
| Oligosaccharide | 100 g |
| Plum concentrate | 2 g |
| Taurine | 1 g |
| Purified water | ad. 900 mL |

The above ingredients are mixed and stirred for about 1 hour while heating at 85 °C accordingly to a commonly employed health drink preparation method. The resulting solution is filtered and put in a sterilized 2-L container. After sealing and sterilization, the solution is kept in a refrigerator and used to prepare a health drink composition of the present disclosure.

The above-described composition is given as a specific example relatively adequate for health drink. However, the composition may be changed variously according to regional or ethnic preferences, such as particular customers, country, purpose of use, or the like.

## Claims

1. A composition containing plant extract from the genus Lindera for use in a treatment of elevated intraocular pressure.

2. The composition for use according to claim 1, wherein the genus *Lindera* comprises *Lindera* erythrocarpa, *Lindera* obtusiloba, *Lindera sericea* and *Lindera* glauca.

3. The composition for use according to 1 or 2, wherein the extract is obtained using an extraction solvent comprising C1-C4 alcohol.

4. A composition containing plant extract froxn the genus Lindera for use in a treatment of glaucoma.

5. The composition for use according to claim 4, wherein the genus Lindera comprises Lindera erythrocarpa, Lindera obtusiloba, Lindera sericea and Lindera glauca.

6. The composition for use according to 4 or 5, wherein the extract is obtained using an extraction solvent comprising C1-C4 alcohol.

7. A health food additive containing a composition containing a plant extract from the genus Lindera for use in lowering elevated intraocular pressure.

8. The health food additive for use according to claim 7, wherein the genus Lindera comprises Lindera erythrocarpa, Lindera obtusiloba, Lindera sericea and Lindera glauca.

9. The health food additive for use according to 7 or 8, wherein the extract is obtained using an extraction solvent comprising C1-C4 alcohol.

10. A health food additive containing a composition containing a plant extract from the genus Lindera for use in treatment of glaucoma.

11. The health food additive for use according to claim 10, wherein the genus Lindera comprises Lindera erythrocarpa, Lindera obtusiloba, Lindera sericea and Lindera glauca.

12. The health food additive for use according to 10 or 11, wherein the extract is obtained using an extraction solvent comprising C1-C4 alcohol.

## Patentansprüche

1. Zusammensetzung, die einen Pflanzenextrakt der Gattung Lindera enthält, zur Anwendung bei der Behandlung von erhöhtem Augeninnendruck.

2. Zusammensetzung zur Anwendung nach Anspruch 1, wobei die Gattung Lindera Lindera erythrocarpa, Lindera obtusiloba, Lindera sericea und Lindera glauca umfasst.

3. Zusammensetzung zur Anwendung nach Anspruch 1 oder 2, wobei der Extrakt mittels eines C1-C4-Alkohol enthaltenden Extraktionslösungsmittels gewonnen ist.

4. Zusammensetzung, die einen Pflanzenextrakt der Gattung Lindera enthält, zur Anwendung bei der Behandlung eines Glaukoms.

5. Zusammensetzung zur Anwendung nach Anspruch 4, wobei die Gattung Lindera Lindera erythrocarpa, Lindera obtusiloba, Lindera sericea und Lindera glauca umfasst.

6. Zusammensetzung zur Anwendung nach Anspruch 4 oder 5, wobei der Extrakt mittels eines C1-C4-Alkohol enthaltenden Extraktionslösungsmittels gewonnen ist.

7. Heilnahrungsadditiv, das eine Zusammensetzung mit einem Pflanzenextrakt der Gattung Lindera enthält, zur Anwendung bei der Senkung von erhöhtem Augeninnendruck.

8. Heilnahrungsadditiv zur Anwendung nach Anspruch 7, wobei die Gattung Lindera Lindera erythrocarpa, Lindera obtusiloba, Lindera sericea und Lindera glauca umfasst.

9. Heilnahrungsadditiv zur Anwendung nach Anspruch 7 oder 8, wobei der Extrakt mittels eines C1-C4-Alkohol enthaltenden Extraktionslösungsmittels gewonnen ist.

10. Heilnahrungsadditiv, das eine Zusammensetzung mit einen Pflanzenextrakt der Gattung Lindera enthält, zur Anwendung bei der Behandlung eines Glaukoms.

11. Heilnahrungsadditiv zur Anwendung nach Anspruch 10, wobei die Gattung Lindera Lindera erythrocarpa, Lindera obtusiloba, Lindera sericea und Lindera glauca umfasst.

12. Heilnahrungsadditiv zur Anwendung nach Anspruch 10 oder 11, wobei der Extrakt mittels eines C1-C4-Alkohol enthaltenden Extraktionslösungsmittels gewonnen ist.

## Revendications

1. Composition contenant un extrait de plante du genre Lindera destinée à être utilisée dans un traitement de la pression intraoculaire élevée.

2. Composition destinée à être utilisée selon la revendication 1, dans laquelle le genre Lindera comprend Lindera erythrocarpa, Lindera obtusiloba, Lindera sericea et Lindera glauca.

3. Composition destinée à être utilisée selon les revendications 1 ou 2, dans laquelle l'extrait est obtenu en utilisant un solvant d'extraction comprenant de l'alcool C1-C4.

4. Composition contenant un extrait de plante du genre Lindera destinée à être utilisée dans un traitement du glaucome.

5. Composition destinée à être utilisée selon la revendication 4, dans laquelle le genre Lindera comprend Lindera erythrocarpa, Lindera obtusiloba, Lindera sericea et Lindera glauca.

6. Composition destinée à être utilisée selon les revendications 4 ou 5, dans laquelle l'extrait est obtenu en utilisant un solvant d'extraction comprenant de l'alcool C1-C4.

7. Additif alimentaire santé contenant une composition contenant un extrait de plante du genre Lindera destiné à être utilisé pour réduire la pression intraoculaire élevée.

8. Additif alimentaire santé destiné à être utilisé selon la revendication 7, dans lequel le genre Lindera comprend Lindera erythrocarpa, Lindera obtusiloba, Lindera sericea et Lindera glauca.

9. Additif alimentaire santé destiné à être utilisé selon les revendications 7 ou 8, dans lequel l'extrait est obtenu en utilisant un solvant d'extraction comprenant de l'alcool C1-C4.

10. Additif alimentaire santé contenant une composition contenant un extrait de plante du genre Lindera destiné à être utilisé dans le traitement du glaucome.

11. Additif alimentaire santé destiné à être utilisé selon la revendication 10, dans lequel le genre Lindera comprend Lindera erythrocarpa, Lindera obtusiloba, Lindera sericea et Lindera glauca.

12. Additif alimentaire santé destiné à être utilisé selon les revendications 10 ou 11, dans lequel l'extrait est obtenu en utilisant un solvant d'extraction comprenant de l'alcool C1-C4.
